# NEW EUROPEAN PATENT SPECIFICATION

(11) **EP 0 215 548 B2**
(45) Date of publication and mention of the opposition decision: **07.01.1998**
(45) Mention of the grant of the patent: 18.08.1993
(21) Application number: 86304970.6
(22) Date of filing: 26.06.1986
(51) Int. Cl.: C12N 15/57, C12P 21/00, C12N 5/10

(54) **Expression of protein C**
Expression von Protein C
Expression de protéine C

(30) Priority: 27.06.1985 US 749600; 15.08.1985 US 766109
(43) Date of publication of application: 25.03.1987
(73) Proprietor: ZYMOGENETICS, INC., Seattle, WA 98105 (US); THE BOARD OF REGENTS OF THE UNIVERSITY OF WASHINGTON, Seattle, Washington 98195 (US)
(72) Inventor: Murray, Mark J., Seattle Washington 98102 (US); Berkner, Kathleen L., Seattle Washington 98199 (US); Foster, Donald C., Seattle Washington 98105 (US); Davie, Earl W., Bellevue Washington 98004 (US)
(74) Representative: Bizley, Richard Edward

(56) References cited:
- EP-A- 191 606
- EP-A- 0 138 222
- Progress in Hematology, vol. XIII, 1983 (New York, London, Paris, San Diego, San Francisco, Sao Paulo, Sydney, Tokyo, Toronto) J.E. Gardiner et al "Human protein C and thromboembolic disease", pages 265-278

## Description

### Technical Field

The present invention relates generally to plasma proteins and DNA sequences encoding them, and more specifically, to the expression of proteins having substantially the same structure and/or activity as human protein C.

### Background Art

Protein C is a zymogen, or precursor, of a serine protease which plays an important role in the regulation of blood coagulation and generation of fibrinolytic activity in vivo. It is synthesized in the liver as a single-chain polypeptide which undergoes considerable processing to give rise to a two-chain molecule comprising heavy (Mr = 40,000) and light (Mr = 21,000) chains held together by a disulphide bond. The circulating two-chain intermediate is converted to the biologically active form of the molecule, known as "activated protein C" (APC), by the thrombin-mediated cleavage of a 12-residue peptide from the amino-terminus of the heavy chain. The cleavage reaction is augmented in vivo by thrombomodulin, an endothelial cell cofactor (Esmon and Owen, Proc. Natl. Acad. Sci. USA 78: 2249-2252, 1981).

Protein C is a vitamin K-dependent glycoprotein which contains approximately nine residues of gamma-carboxyglutamic acid (Gla) and one equivalent of beta-hydroxyaspartic acid which are formed by post-translational modifications of glutamic acid and aspartic acid residues, respectively. The post-translational formation of specific gamma-carboxyglutamic acid residues in protein C requires vitamin K. These unusual amino acid residues bind to calcium ions and are believed to be responsible for the interaction of the protein with phospholipid, which is required for the biological activity of protein C.

In contrast to the coagulation-promoting action of other vitamin K-dependent plasma proteins, such as factor VII, factor IX, and factor X, activated protein C acts as a regulator of the coagulation process through the inactivation of factor Va and factor Vllla by limited proteolysis. The inactivation of factors Va and Villa by protein C is dependent upon the presence of acidic phospholipids and calcium ions. Protein S has been reported to regulate this activity by accelerating the APC-catalyzed proteolysis of factor Va (Walker, J. Biol, Chem. 255: 5521-5524, 1980).

Protein C has also been implicated in the action of tissue-type plasminogen activator (Kisiel and Fujikawa, Behring Inst. Mitt. 73: 29-42, 1983). Infusion of bovine APC into dogs results in increased plasminogen activator activity (Comp and Esmon, J. Clin. Invest. 68: 1221-1228, 1981). Recent studies (Sakata et al., Proc. Natl. Acad. Sci. USA 82: 1121-1125, 1985) have shown that addition of APC to cultured endothelial cells leads to a rapid, dose-dependent increase in fibrinolytic activity in the conditioned media, reflecting increases in the activity of both urokinase-related and tissue-type plasminogen activators by the cells. APC treatment also results in a dose-dependent decrease in antiactivator activity.

Inherited protein C deficiency is associated with recurrent thrombotic disease (Broekmans et al., New Eng. J. Med. 309: 340-344, 1983; and Seligsohn et al., New Eng. J. Med. 310: 559-562, 1984) and may result from genetic disorder or from trauma, such as liver disease or surgery. This condition is generally treated with oral anti-coagulants. Beneficial effects have also been obtained through the infusion of protein C-containing normal plasma (see Gardiner and Griffin in Prog. in Hematology, ed. Brown, Grune & Stratton, Ny, 13: 265-278). In addition, some investigators have discovered that the anti-coagulant activity of protein C is useful in treating thrombotic disorders, such as venous thrombosis (WO 85/00521). In some parts of the world, it is estimated that approximately 1 in 16,000 individuals exhibit protein C deficiency. Further, a total deficiency in protein C is fatal in newborns.

While natural protein C may be purified from clotting factor concentrates (Marlar et al., Blood 59: 1067-1072) or from plasma (Kisiel, ibid), it is a complex and expensive process, in part due to the limited availability of the starting material and the low concentration of protein C in plasma. Furthermore, the therapeutic use of products derived from human blood carries the risk of disease transmission by, for example, hepatitis virus, cytomegalovirus, or the causative agent of acquired immune deficiency syndrome (AIDS). In view of protein C's clinical applicability in the treatment of thrombotic disorders, the production of useful quantities of protein C and activated protein C is clearly invaluable.

EP-A-191606, published on 20.8.1986, discloses DNA sequences encoding human protein C.

### Disclosure of Invention

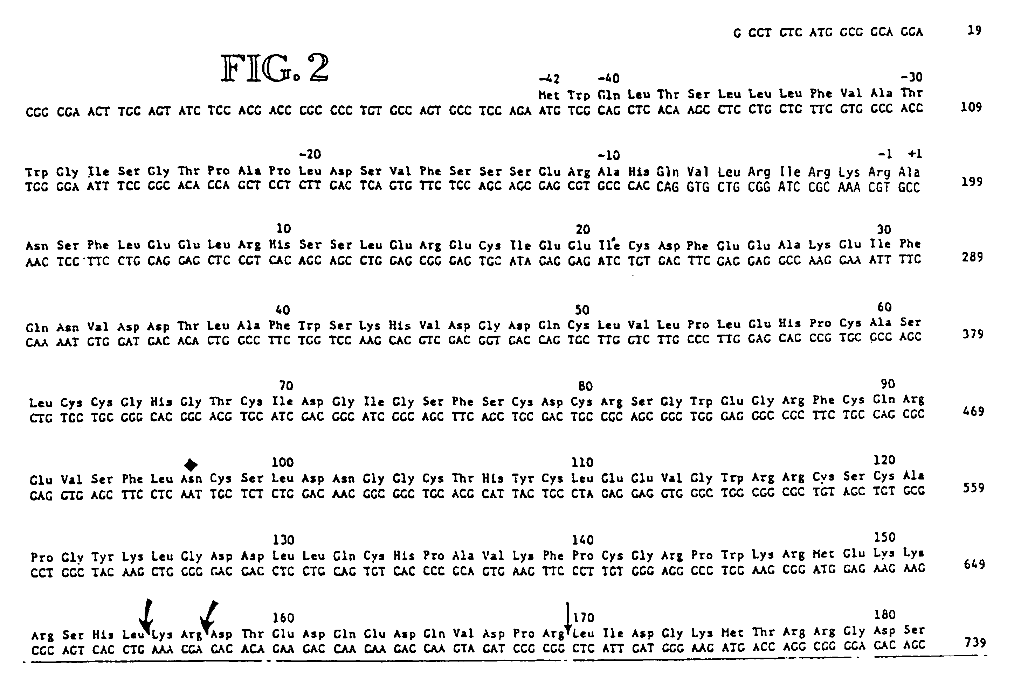

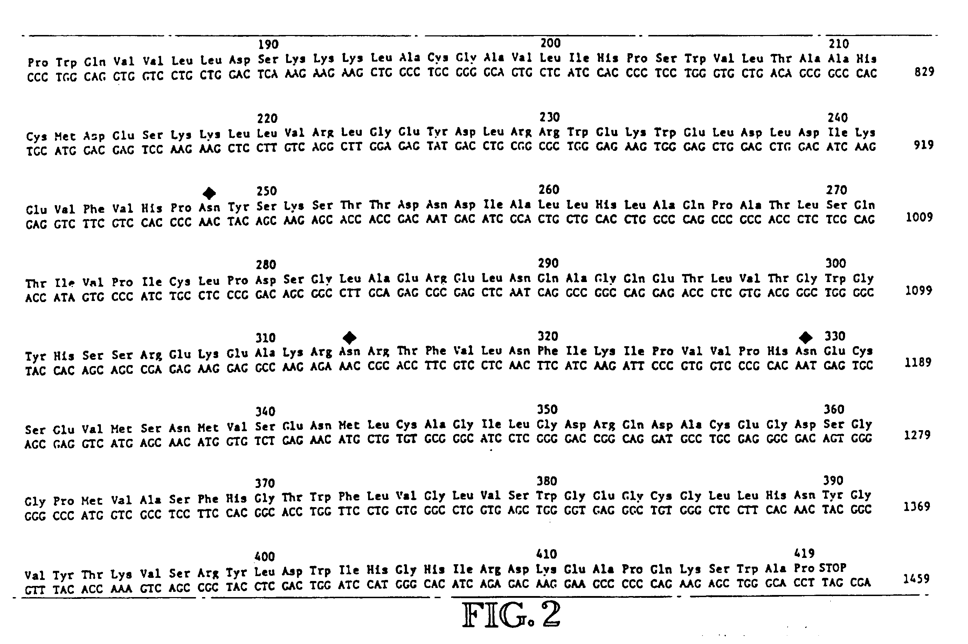

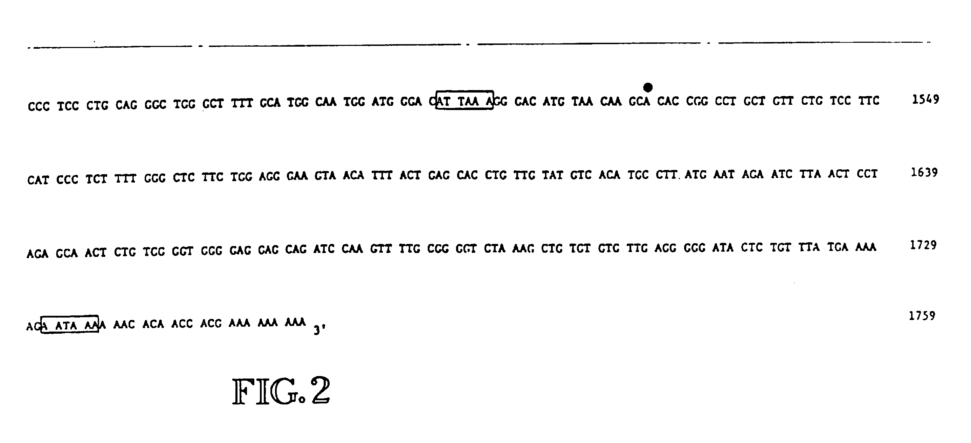

Briefly stated, the present invention discloses a DNA sequence which codes for a protein which upon activation by proteolytic processing in the secretion pathway results in a human activated protein C, said DNA sequence not including a sequence encoding the twelve amino acid activation peptide shown in Figure 2, said activated protein C having an amino acid sequence shown in Figure 2. The invention also provides a recombinant expression vector comprising a DNA sequence which codes for a protein which upon activation by proteolytic processing in the secretion pathway results in a human activated protein C, said DNA sequence not including a sequence encoding the twelve amino acid activation peptide shown in Figure 2, said activated protein C having an amino acid sequence shown in Figure 2. In addition, the present invention discloses an expression vector capable of integration in mammalian host cell DNA, said expression vector including a promoter followed downstream by a nucleotide sequence of claim 2, said nucleotide sequence being followed downstream by a polyadenylation signal, wherein transcription of the nucleotide sequence is directed by the promoter. In one embodiment,the expression vector includes a selectable marker located between the nucleotide sequence and the polyndenylation signal, transcription of the selectable marker being directed by the promoter. The expression vector may also include a set of RNA splice sites.

A related aspect of the present invention discloses mammalian cells transfected to express human activated protein C. The mammalian cells are transfected with an expression vector as defined above. Within one embodiment, a selectable marker is also introduced into the cells and stably transfected cells are selected.

A further aspect of the invention discloses a method for producing human activated protein C. The method comprises introducing into a mammalian host cell an expression unit comprising a sequence of claim 1, which encodes a protein which upon activation by intracellular proteolytic processing results in a human activated protein C; growing said mammalian host cell in an appropriate medium which contains vitamin K; and isolating the protein product encoded by said expression unit and produced by said mammalian host cell.

The proteins produced within the present invention may be used as active therapeutic substances, including the regulation of blood coagulation. Further, these proteins may be combined with a physiologically acceptable carrier and/or diluent to provide suitable pharmaceutical compositions.

Other aspects of the invention will become evident upon reference to the detailed description and attached drawings.

### Brief Description of the Drawings

Figure 1 is a partial restriction map of the protein C cDNA in pHCλ6L. The coding region is indicated by an open box.

Figure 2 illustrates the nucleotide sequence of the complete protein C cDNA and the deduced amino acid sequence of protein C. Arrows indicate cleavage sites for removal of the connecting dipeptide and activation peptide.

Figure 3 illustrates a restriction enzyme map of the genomic DNA coding for human protein C. Numbers below the line indicate length in kilobases (kb).

Figure 4 illustrates the complete genomic sequence, including exons and introns of the human protein C gene. Arrowheads indicate intron-exon splice junctions. The polyadenylation or processing sequences of A-T-T-A-A-A and A-A-T-A-A-A at the 3' end are boxed. ◆, potential carbohydrate attachment sites: ·⃝, apparent cleavage sites for processing of the connecting dipeptide; ↓, site of cleavage in the heavy chain when protein C is converted to activated protein C; •, sites of polyadenylation.

Figure 5 illustrates a schematic two dimensional model for the structure of human protein C.

Figure 6 illustrates the subcloning of the 5' and 3' portions of a protein C partial cDNA clone.

Figure 7 illustrates the removal of intron A from the genomic clone, resulting in the fusion of exons I and II.

Figure 8 illustrates the fusion of exons I and II to the 5'-most portion of the cDNA insert of Figure 1.

Figure 9 illustrates the construction of a plasmid comprising the complete coding sequence for protein C.

Figure 10 illustrates the expression vector pD7C. Symbols used are ori, the adenovirus 5 0-1 map unit sequence; E, the SV40 enhancer; Ad2MLP, the adenovirus 2 major late promoter; L 1-3, the adenovirus 2 tripartite leader; 5'ss, 5' splice site; 3'ss, 3' splice site; pA, the SV40 early polyadenylation signal; and Δ. the deleted region of the pBR322 "poison" sequences.

Figure 11 illustrates the expression vector pD5(PC-DHFR'). DHFR' denotes the methotrexate resistant mutant dihydrofolate reductase gene sequence; pA denotes the SV40 late polyadenylation signal. Other symbols used are as described for Figure 7.

Figure 12 illustrates the expression vector pDX/PC. Symbols used are as described for Figure 11.

### Best Mode for Carrying Out the Invention

Prior to setting forth the invention, it may be helpful to an understanding thereof to set forth definitions of certain terms to be used hereinafter.

Biological Activity: A function or set of functions performed by a molecule in a biological context (i.e., in an organism or an in vitro facsimile). Biological activities of proteins may be divided into catalytic and effector activities. Catalytic activities of the vitamin K-dependent plasma proteins generally involve the specific proteolytic cleavage of other plasma proteins, resulting in activation or deactivation of the substrate. Effector activities include specific binding of the biologically active molecule to calcium or other small molecules, to macromolecules, such as proteins, or to cells. Effector activity frequently augments, or is essential to, catalytic activity under physiological conditions.

For protein C, biological activity is characterized by its anticoagulant and fibrinolytic properties. Protein C, when activated, inactivates factor Va and factor VIlla in the presence of phospholipid and calcium. Protein S appears to be involved in the regulation of this function (Walker, ibid). Activated protein C also enhances fibrinolysis, an effect believed to be mediated by the lowering of levels of plasminogen activator inhibitors (van Hinsbergh et al., Blood 65: 444-451, 1985). As more fully described below, that portion of protein C encoded by exons VII and VIII of the protein C gene are primarily responsible for its catalytic activities.

Pre-pro peptide: An amino acid sequence which occurs at the amino terminus of some proteins and is generally cleaved from the protein during translocation. Pre-pro peptides comprise sequences directing the protein into the secretion pathway of the cell (signal sequences) and are characterised by the presence of a core of hydrophobic amino acids. They may also comprise processing signals. As used herein, the term "pre-pro peptide" may also mean a portion of the naturally occuring pre-pro peptide.

Expression Unit: A DNA construct comprising a primary nucleotide sequence encoding a protein of interest, together with other nucleotide sequences which direct and control the transcription of the primary nucleotide sequence. An expression unit consists of at least the primary nucleotide sequence and a promoter sequence located upstream from and operably linked to the primary nucleotide sequence and a polyadenylation signal located downstream. Additional genetic elements may also be included to enhance efficiency of expression. These elements include enhancer sequences, leaders, and mRNA splice sites.

Expression Vector: A DNA molecule which contains, inter alia, a DNA sequence encoding a protein of interest together with a promoter and other sequences which facilitate expression of the protein. Expression vectors further contain genetic information which provides for their replication in a host cell. Examples of expression vectors commonly used for recombinant DNA are plasmids and certain viruses, although they may contain elements of both. They also may include a selectable marker.

As noted above, protein C is produced in the liver and requires vitamin K for its biosynthesis. Vitamin K is necessary for the formation of specific gamma-carboxy-glutamic acid residues in the amino-terminal region of the light chain. These amino acid residues are formed by a post-translational modification, and are required for calcium-mediated binding to phospholipid. In addition, protein C contains one beta-hydroxyaspartic acid residue which as also formed in a post-translational modification. However, the role of this amino acid residue is not known.

Given the fact that the activity of protein C is dependent upon post-translational modifications involving the gamma carboxylation of specific glutamic acid residues, and may also be dependent upon the hydroxylation of a specific aspartic acid residue, it is unlikely that an active product could be produced through the cloning and expression of protein C in a microorganism.

The present invention enables a method for producing a protein which is gamma-carboxylated and has the biological activity of human activated protein C without the necessity for activation.

The light and heavy chains of bovine protein C have been sequenced (Fernlund and Stenflo, J. Biol. Chem. 257: 12170-12179, 1982; and Stenflo and Fernlund. J. Biol, Chem, 257: 12180-12190, 1982). Isolation and characterization of human protein C have been described by Kisiel, J. Clin. Invest. 64: 761-769, 1979. With the exception of the amino-terminal aspartic acid residue, the amino-terminal sequence of the heavy chain of human protein C is different from the first eighteen amino acids found in the heavy chain of the bovine protein. The anticoagulant activities of both the human and bovine enzymes were found to be highly species specific. Species specificity is believed to be mediated by protein S (Walker, Thromb. Res. 22: 321-327, 1981). However, the human and bovine proteins show considerable overall structural homology to each other and to other vitamin K-dependent plasma proteins, including prothrombin, factor VII, factor IX, and factor X. Similarities include the presence of the Gla residues in the light chain and the active site serine in the heavy chain, as well as other amino acid sequence homology in the amino-terminal region of the light chain.

Within the present invention, a λgtll cDNA library was prepared from human liver mRNA. This library was then screened with ¹²⁵I labeled antibody to human protein C. Antibody-reactive clones were further analyzed for the synthesis of a fusion protein of β-galactosidase and protein C in the λgtll vector.

One of the clones gave a strong signal with the antibody probe and was found to contain an insert of approximately 1400 bp. DNA sequence analysis of the DNA insert revealed a predicted amino acid sequence which shows a high degree of homology to major portions of the bovine protein C, as determined by Fernlund and Stenflo (J. Biol. Chem. 257: 12170-12179; J. Biol. Chem. 257: 12180-12190).

The DNA insert contained the majority of the coding region for protein C beginning with amino acid 64 of the light chain, including the entire heavy chain coding region, and proceeding to the termination codon. Further, following the stop codon of the heavy chain, there were 294 base pairs of 3' noncoding sequence and a poly (A) tail of 9 base pairs. The processing or polyadenylation signal A-A-T-A-A-A was present 13 base pairs upstream from the poly (A) tail in this cDNA insert. This sequence was one of two potential polyadenylation sites.

The cDNA sequence also contained the dipeptide Lys-Arg at position 156-157, which separates the light chain from the heavy chain and is removed during processing by proteolytic cleavage resulting in secretion of the two chain molecule. Upon activation by thrombin, the heavy chain of human protein C is cleaved between arginine- 169 and leucine-170, releasing the activation peptide (Figure 2).

By a similar method, a second cDNA which lacked the coding sequence for the pre-pro peptide and the first 23 amino acids of protein C was isolated. Using this cDNA as a hybridization probe, the remainder of the coding sequence was obtained from a human genomic DNA library in λ Charon 4A (Foster et al., Proc. Natl. Acad. Sci. USA 82: 4673- 4677, 1985). Three different λ Charon 4A phage were isolated that contained overlapping inserts for the protein C gene.

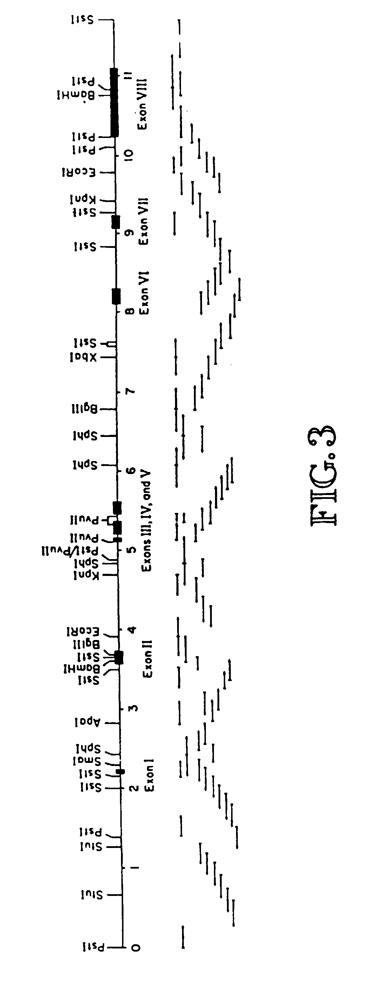

The positions of exons on the three phage clones were determined by Southern blot hybridization of digests of these clones with probes made from the 1400 bp cDNA described above. The genomic DNA inserts in these clones were mapped by single and double restriction enzyme digestion followed by agarose gel electrophoresis, Southern blotting, and hybridization to radiolabeled 5' and 3' probes derived from the cDNA for human protein C, as shown in Figure 3.

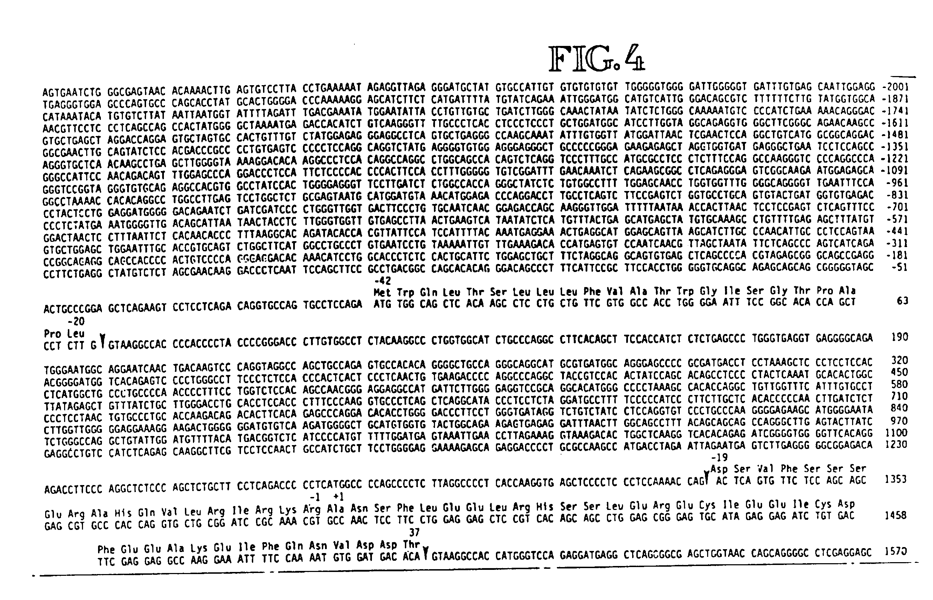

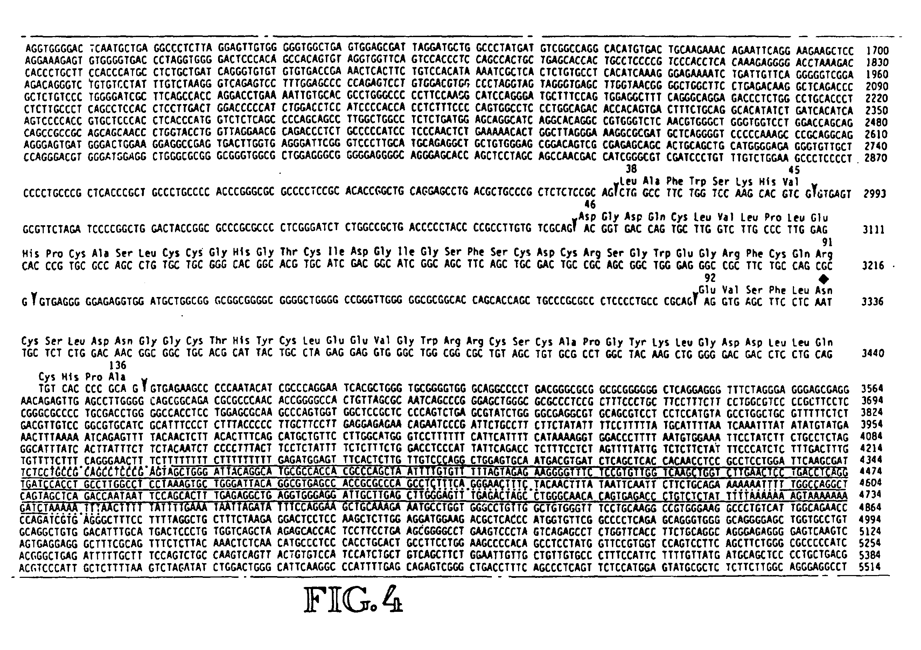

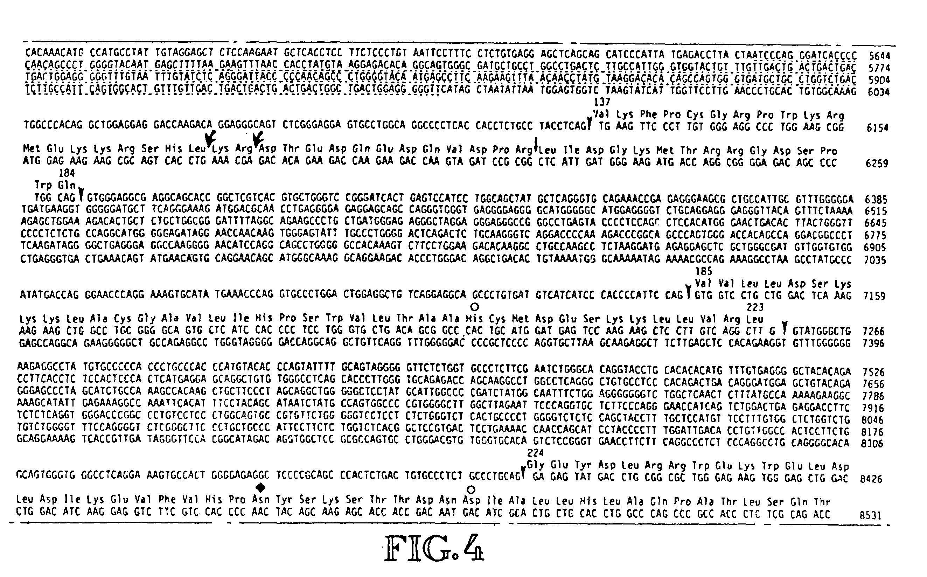

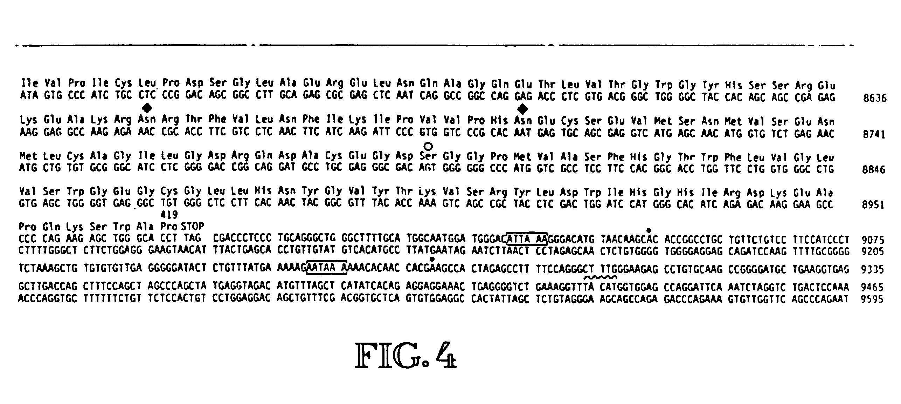

DNA sequencing studies were performed using the dideoxy chain-termination method. As shown in Figure 4, the nucleotide sequence for the gene for human protein C spans approximately 11 kb of DNA. These studies further revealed a potential pro-pro peptide of 42 amino acids. Based on homology with the pre-pro peptide of bovine protein C in the region -1 to -20, it is likely that the pre-pro sequence is cleaved by a signal peptidase following the Ala residue at position -10. Processing to the mature protein involves additional proteolytic cleavage following residue -1 to remove the amino-terminal propeptide, and at residues 155 and 157 to remove the Lys-Arg dipeptide which connects the light and heavy chains. This final processing yields a light chain of 155 amino acids and a heavy chain of 262 amino acids.

The protein C gene is composed of eight exons ranging in size from 25 to 885 nucleotides, and seven introns ranging in size from 92 to 2668 nucleotides. Exon I and a portion of exon II code for the 42 amino acid pre-pro peptide. The remaining portion of exon II, exon III, exon IV, exon V, and a portion of exon VI code for the light chain of protein C. The remaining portion of exon VI, exon VII, and exon VIII code for the heavy chain of protein C. The amino acid and DNA sequences for a cDNA coding for human protein C are shown in Figure 2.

The location of the introns in the gene for protein C are primarily between various functional domains. Exon II spans the highly conserved region of the pre-pro peptide and the gamma-carboxyglutamic acid (Gla) domain. Exon III includes a stretch of eight amino acids which connect the Gla and growth factor domains. Exons IV and V each represent a potential growth factor domain, while exon VI covers a connecting region which includes the activation peptide. Exons VII and VIII cover the catalytic domain typical of all serine proteases.

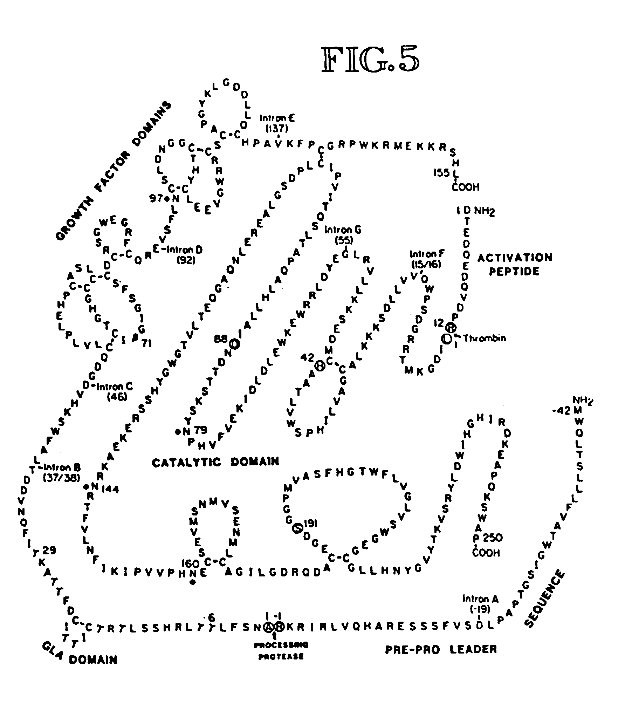

The amino acid sequence and tentative structure for human pre-pro protein C are shown in Figure 5. Protein C is shown without the Lys-Arg dipeptide, which connects the light and heavy chains. The location of the seven introns (A through G) is indicated by solid bars. Amino acids flanking known proteolytic cleavage sites are circled, ◆ designates potential carbohydrate binding sites. The first amino acid in the light chain, activation peptide, and heavy chain start with number 1. This numbering differs from that shown in Figures 2 and 4.

Carbohydrate attachment sites are located at residue 97 in the light chain and residues 79, 144, and 160 in the heavy chain, according to the numbering scheme of Figure 5. The carbohydrate moiety is covalently linked to Asn. In the majority of instances, the carbohydrate attachment environment can be represented by Asn-X-Ser or Asn-X-Thr, where x = any amino acid.

As noted above, protein C plays a regulatory role in the coagulation process. The catalytic domain, encoded by exons VII and VIII, possesses serine protease activity which specifically cleaves certain plasma proteins (i.e., factors Va and VIIIa), resulting in their activation or deactivation. As a result of this selective proteolysis, protein C displays anticoagulant and fibrinolytic activities.

Due to the presence of intervening sequences in the genomic clone, merely joining the genomic and cDNA sequences to provide a complete coding sequence is not sufficient for constructing an acceptable expression unit. It is therefore necessary to delete those intervening sequences for reasons more fully described below if a genomic clone is used to construct the expression unit.

The 5' coding region may also be obtained by alternative methods and consequently eliminate the need to delete intervening sequences. The 5' coding region may be obtained by using probes derived from the existing cDNA or genomic clones to probe additional libraries. By this method, a full-length cDNA was isolated. Furthermore, the amino-terminal portions of the vitamin K-dependent plasma proteins are responsible for their respective calcium binding activities: It has been found that, as a result of this functional homology, the calcium binding domains of these molecules may be interchanged and still retain the activity specific to the catalytic domain of the resultant molecule. For example, as described in U.S. Patent Application serial No. 724,311, filed April 17, 1985, the amino-terminal portion (calcium binding domain) of factor IX may be joined to factor VII at amino acid 36 to produce a protein having the activity of factor VII. Factor VII, factor IX, factor X, prothrombin, and protein S share this amino-terminal sequence homology with protein C. Consequently, a cloned sequence comprising the 5'-coding region of the gene for any of these proteins might be substituted for the corresponding sequence of the protein C gene. Additionally, suitable coding sequences may be synthesized based on the known amino acid sequences of several of the vitamin K-dependent plasma proteins or on the sequence of the genomic protein C exons disclosed herein. Techniques for producing synthetic nucleotide sequences are well known in the art. For example, a set of overlapping oligonucleotides may be synthesized and annealed in pairs to yield doubles-tranded fragments with overlapping cohesive termini. These fragments are then ligated as any restriction fragments would be. The resultant synthetic fragment is then ligated to the cDNA at a convenient restriction site. The junction sequence may be modified as necessary by oligonucleotide-directed mutagenesis.

When clones representing the entire coding sequence have been obtained, the appropriate regions may be joined, as necessary, to generate the desired coding sequence. Fragments obtained from one or more libraries are cut with appropriate restriction endonucleases and joined together enzymatically in the proper orientation. Depending on the fragments and the particular restriction endonucleases chosen, it may be necessary to remove unwanted DNA sequences through a "loop out" process of deletion mutagenesis or through a combination of restriction endonuclease cleavage and mutagenesis. The sequence so obtained should preferably be in the form of a continuous open reading frame, that is, that it lack the intervening sequences (introns) generally found in higher eukaryotic genes. The presence of introns in cloned genes may lead to aberrant splicing of messenger RNA and/or reduced efficiency of gene expression or instability upon amplification when the gene sequence is introduced into a mammalian host cell. It is preferred that this coding sequence further encode a pre-pro peptide in order to facilitate proper processing and secretion of the protein C. The pre-pro peptide may be that of protein C or another secreted protein, such as factor IX, factor VII, or prothrombin.

it is however, desirable to produce active protein C directly, thereby removing the need to activate the protein product either in vitro or in vivo. The cleavage sites involved in the maturation and activation of protein C are known (Foster and Davle, ibid). A sequence encoding APC may be constructed by deleting the region encoding the activation peptide through oligonucleotide directed deletion mutagenesis. The resultant protein will then be activated by proteolytic processing in the secretion pathway.

The coding sequence for activated protein C is then inserted into a suitable expression vector which is, in turn, used to transfect a mammalian cell line. Expression vectors for use in carrying out the present invention will comprise a promotor capable of directing the transcription of a foreign gene introduced into a mammalian cell. Viral promoters are preferred due to their efficiency in directing transcription. A particularly preferred promotor is the major late promoter from adenovirus 2. Such expression vectors will also preferably contain a set of RNA splice sites located downstream from the promoter and upstream from the insertion site for the protein C sequence or within the protein C sequence itself. Preferred RNA splice sites may be obtained from adenovirus and/or Immunoglobulin genes. Also contained In the expression vectors is a polyadenylation signal, located downstream of the insertion site. Viral polyadenylation signals are particularly preferred, such as the early or late polyadenylation signals from SV40 or the polyadenylation signal from the adenovirus 5 Elb region. In a particularly preferred embodiment, the expression vector also comprises a noncoding viral leader sequence, such as the adenovirus 2 tripartite leader, located between the promoter and the RNA splice sites. Preferred vectors may also include enhancer sequences, such as the SV40 enhancer and the sequences encoding the adenovirus VA RNAs.

Cloned gene sequences may then be introduced into cultured mammalian cells by, for example, calcium phosphate-mediated transfection (Wigler et al., Cell 14: 725, 1978; Corsaro and Pearson, Somatic Cell Genetics 7: 603, 1981; Graham and Van der Eb, Virology 52: 456, 1973). A precipitate is formed of the DNA and calcium phosphate, and this precipitate is applied to the cells. Some of the cells take up the DNA and maintain it inside the cell for several days. A small fraction of these cells (typically 10⁻⁴) integrate the DNA into the genome. In order to identify these integrants, a gene that confers a selectable phenotype (a selectable marker) is generally introduced into the cells along with the gene of interest. Preferred selectable markers include genes that confer resistance to drugs, such as neomycin, hygromycin, and methotrexate. Selectable markers may be introduced into the cell on a separate plasmid at the same time as the gene or interest, or they may be introduced on the same plasmid. If on the same plasmid, the selectable marker and the gene of interest may be under the control of different promoters or the same promoter. In one embodiment, the selectable marker is placed on the same plasmid with the sequence encoding protein C such that both sequences are controlled by the same promoter, an arrangement known as a dicistronic message. Constructs of this type are known in the art (for example, European Patent Office publication 117,058), It may also be advantageous to add additional DNA, known as "carrier DNA," to the mixture which is introduced into the cells. After the cells have taken up the DNA, they are allowed to grow for a period of time, typically 1-2 days, to begin expressing the gene of interest. Drug selection is then applied to select for the growth of cells which are expressing the selectable marker in a stable fashion. Clones of such cells may be screened for expression of protein C.

The copy number of the integrated gene sequence may be increased through amplification by using certain selectable markers (e.g., dihydrofolate reductase, which confers resistance to methotrexate). The selectable marker is introduced into the cells along with the gene of interest, and drug selection is applied. The drug concentration is then increased in a stop-wise manner, with selection of resistant cells at each step. By selecting for increased copy number of cloned sequences, expression levels of the encoded protein may be substantially increased.

Protein C produced according to the present invention may be purified by a modification of the method of Kisiel and Davie (ibid). Medium containing protein C is mixed with sodium citrate and barium chloride and the precipitate collected. The precipitate is washed, redissolved and reprecipitated with ammonium sulfate, then dissolved in sodium phosphate-benzamidine, dialyzed, and applied to a DEAE-Sephadex A-50 column. The protein C-containing peak, which also contains prothrombin, is further purified by affinity chromatography on heparin-agarose (Comp and Esmon, Blood 54: 1272, 1979) or by immunoadsorption.

To summarize the Examples which follow, Example 1 describes the cloning of DNA sequences encoding human protein C. Example 2 describes the construction of a full-length coding sequence for protein C from the sequences isolated in Example 1. Example 3 describes the construction of expression vectors for the protein C DNA. Example 4 describes the production of protein C using transfected mammalian cells. Example 5 describes a full-length cDNA encoding protein C and its expression in transfected mammalian cells. These Examples although not directly examples of the claimed invention are useful guidance for practising the claimed invention.

### EXAMPLES

Restriction endonucleases and other DNA modification enzymes (e.g., T₄ polynucleotide kinase, calf alkaline phosphatase, Klenow DNA polymerase, T₄ polynucleotide ligase) were obtained from Bethesda Research Laboratories (BRL) and New England Biolabs and are used as directed by the manufacturer, unless otherwise noted.

Oligonucleotides may be synthesized on an Applied Biosystems Model 380 A DNA synthesizer and purified by polyacrylamide gel electrophoresis on denaturing gels. E. coli cells may be transformed as described by Maniatis et al. (Molecular Cloning: A Laboratory Manual, Cold Spring Harbor Laboratory, 1982). Ml3 and pUC cloning vectors and host strains were obtained from BRL.

### Example 1

### Cloning of DNA Sequences Encoding Human Protein C

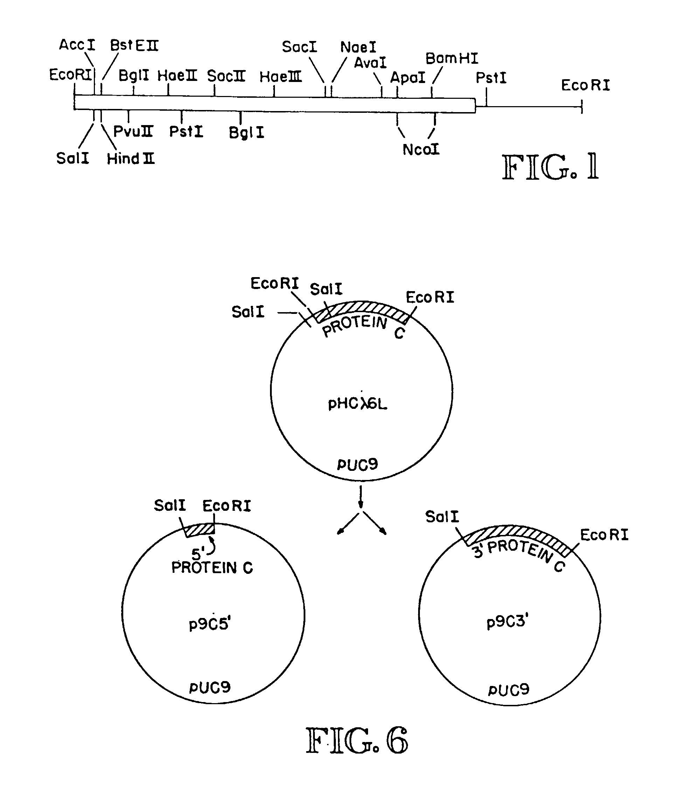

A cDNA coding for a portion of human protein C was prepared as described by Foster and Davie (ibid). Briefly, a λgtll cDNA library wan prepared from human liver mRNA by conventional methods. Clones were screened using ¹²⁵I.labeled affinity-purified antibody to human protein C, and phage were prepared from positive clones by the plate lysate method (Maniatis et al., ibid), followed by banding on a cesium chloride gradient. The cDNA inserts were removed using Eco RI and subcloned into plasmid pUC9 (Vieira and Messing, Gene 19: 259-268, 1982). Restriction fragments were subcloned in the phage vectors Ml3mpl0 and ml3mpll (Messing, Meth. in Enzymology 101: 20-77, 1983) and sequenced by the dideoxy method (Sanger et al., Proc. Natl. Acad. Sci. USA 74: 5463-5467, 1977). A clone was selected which contained DNA corresponding to the known sequence of human protein C (Kisiel, ibid) and encoded protein C beginning at amino acid 64 of the light chain and extending through the heavy chain and into the 3' non-coding region. This clone was designated XHC1375. A second cDNA clone coding for protein C from amino acid 24 was identified. The insert from this clone was subcloned into pUC9 and the plasmid designated pHCλ6L (Figure 1). This clone encodes a major portion of protein C, including the heavy chain coding region, termination codon, and 3' non-coding region.

The cDNA insert from λHCl375 was nick translated using α-³²P dNTP's and used to probe a human genomic library in phage X Charon 4A (Maniatis et al., Cell 15: 687-702, 1978) using the plaque hybridisation procedure of Benton and Davis (Science 196: 181-182, 1977) as modified by Woo (Meth. in Enzymology 68: 381-395, 1979). Positive clones were isolated and plaque-purified (Foster et al., Proc. Natl. Acad. Sci. USA 82: 4673-4677, 1985, herein incorporated by reference). Phage DNA prepared from positive clones (Silhavy et al., in Experiments with Gene Fusion, Cold Spring Harbor Laboratory, 1984) was digested with Eco RI or Bgl II and the genomic inserts purified and subcloned in pUC9. Insert restriction fragments were subcloned into Ml3 vectors and sequenced to confirm their identity and establish the DNA sequence of the entire gene.

The cDNA insert of pHCX6L was nick translated and used to probe the phage λ Charon 4A library. One genomic clone was identified which hybridized to probes made from the 5' and 3' ends of the cDNA. This phage clone was digested with Eco RI and a 4.4 kb fragment, corresponding to the 5' end of the protein C gene, was subcloned into pUC9. The resultant recombinant plasmid was designated pHCR4.4. Complete DNA sequence analysis revealed that the insert in pHCR4.4 comprised two exons of 70 and 167 base pairs separated by an intron of 1263 bp. The first exon encodes amino acids -42 to -19; the second encodes amino acids -19 to 37. Sequence analysis confirmed the DNA sequence of the entire protein C gene.

As noted above, it is then necessary to remove the intron in order to use a genomic clone to construct an acceptable coding sequence for use within the present invention.

### Example 2

### Construction of a Full-Length Coding Sequence for Protein C

A full-length coding sequence for Protein C, including the pre-pro peptide, is constructed by joining the appropriate fragments of the cDNA and genomic clones. This is accomplished by removing the intron from the genomic clone (pHCR4.4) end joining the fused exons to the cDNA (from pHCλ6L) at convenient restriction sites. The desired genomic:cDNA junction is then generated by looping out unwanted sequences by oligonucleotide-directed deletion mutagenesis.

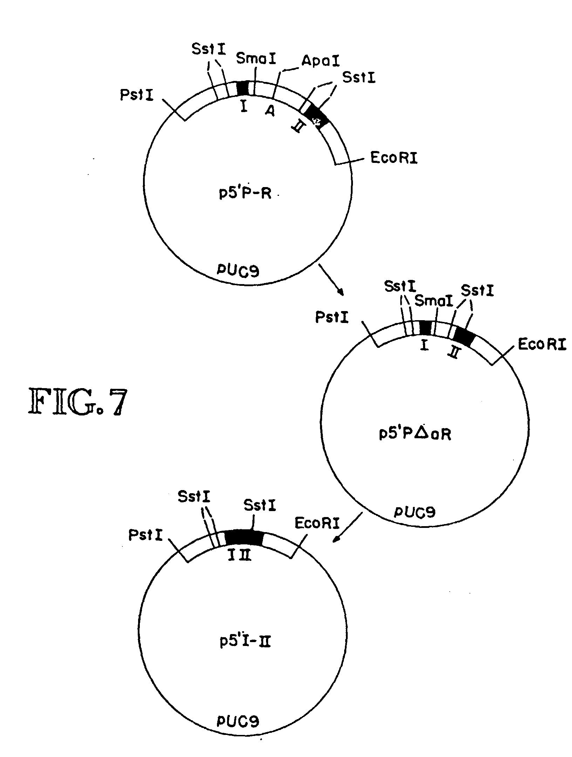

Plasmid pHCλ6L contains the protein C partial cDNA cloned in the Eco RI site of pUC9 (Figure 1). The cDNA insert is subcloned in two fragments to prepare it for joining to the 5'-most coding region from the genomic clone. Plasmid pHCλ6L is digested with Eco RI and Sal I, and the reaction mixture is then extracted with phenol and CHCl₃ and ethanol-precipitated. The resulting DNA fragments are resuspended in ligation buffer, and T₄ DNA ligase is added. The ligation mixture is incubated at 15°C for 14 hours. An aliquot of the ligation mix is used to transform E. coli JM83, and the cells are plated on LB agar containing X-gal. White colonies are selected, and plasmid DNA is prepared. The DNA is analyzed by restriction enzyme digestion to identify clones containing the 3' portion of the cDNA (ca. 1450 bp insert) and the 5' portion of the cDNA (ca. 65 bp insert). These clones are designated p9C3' and p9C5', respectively (Figure 6).

The 5' coding region missing from the cDNA is contained in exons I and II of the genomic clone pHCR4.4. This plasmid contains an insert of approximately 4400 base pairs and terminates on its 3' end at an Eco RI site located in intron B.

To remove the coding sequences from pHCR4.4, the plasmid is digested with Pstl and Eco RI and the resulting fragments separated by electrophoresis in an agarose gel. The ca.2540 bp fragment containing exons I and II is isolated from the gel and extracted with CTAB (Langridge, et al., Analyt. Biochem. 103: 264, 1980). This fragment, designated 5'P-R, is subcloned into pUC9 to produce plasmid p5'P-R (Figure 7).

The intron in p5'P-R (designated intron A), is removed in a two-step process (Figure 7). The plasmid is digested with Apa I, which cleaves at a unique site in the intron and leaves 3' overhanging ends. The linearized plasmid is then treated with Bal 3l exonuclease or T₄ polymerase to remove approximately 400 bp from each end and the resultant fragment ends are blunted with Sl nuclease. The linearized plasmid is recircularised with ligase and used to transform E. coli JM83. Plasmid DNA is extracted and analyzed for the presence of the Sma I and Sat I restriction sites in intron A, and a plasmid having a Sma l-Sstl fragment reduced to 300-400 bp is chosen and designated p5'PΔaR.

The remainder of intron A is removed by oligonucleotide-directed deletion mutagenesis, essentially as described by Zoller and Smith (Manual for Advanced Techniques in Molecular Cloning Course, Cold spring Harbor Laboratory, 1983) for the two-primer method. p5'PΔaR is digested with Pst I and Eco RI, and the protein C fragment is subcloned into Pst I + Eco RI-digested Ml3mp9. Plus strand phage DNA is prepared as template and annealed to oligonucleotide mut-l (Table 1). This mutagenic oligonucleotide comprises sequences complementary to the exon I and II sequences to be joined. The Ml3 universal sequencing primer is annealed 3'to mut-1 on the same template. The primers are extended using DNA polymerase I (Klenow fragment) and nucleoside triphosphates in the presence of T₄ ligase. The resulting duplex DNA circles are transformed into E. coli JMl03 and the resulting plaques screened under stringent hybridization conditions using the ³²P-labeled mutagenic oligonucleotide as probe. DNA from positive plaques is isolated and sequenced using oligonucleotide primer-1 (Table 1), which primes in exon II, allowing the determination of the DNA sequence across the deletion junction. A molecule having the correct inframe fusion of exons I end II is selected. The Pstl-EcoRI fragment is isolated from the Ml3 replicative form by restriction endonuclease digestion and agarose gel electrophoresis and is subcloned into pUC9 to produce plasmid p5'l-ll (Figure 7).

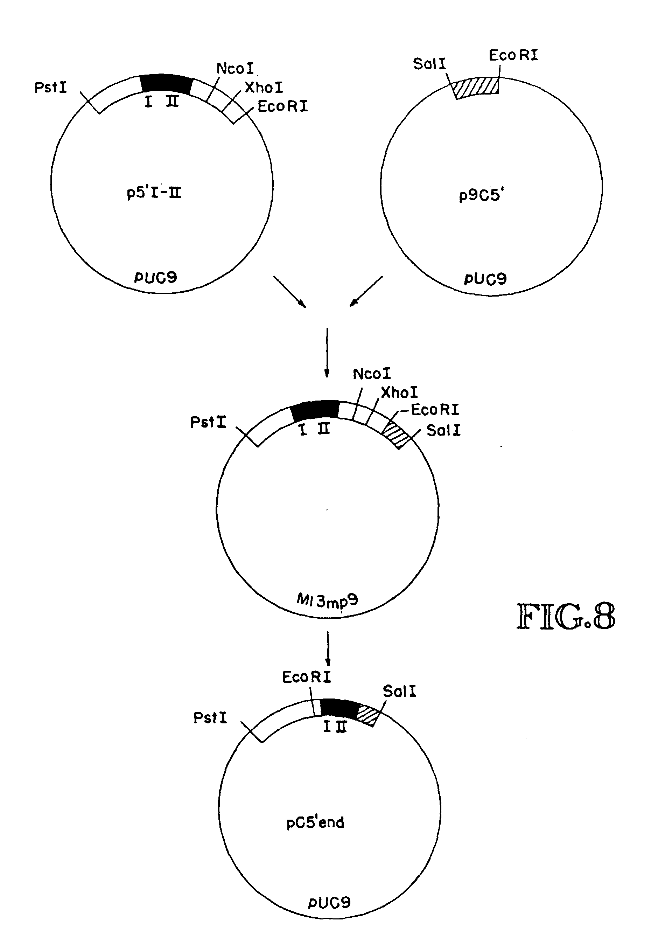

Referring to Figure 8, to join the 5' coding region to the cDNA, the ca. 1277 bp Pst I.Eco RI fragment of p5'l-ll is isolated from a Pst I + Eco RI digest of the plasmid and purified by agarose gel electrophoresis. The 65 bp 5'-most cDNA fragment is isolated from a Sal I + Eco RI digest of p9C5' and purified by electrophoresis on an acrylamide gel. The two fragments are ligated at their Eco RI termini, and the resulting ca. 1330 bp Pst I-Sal I fragment is subcloned into pst I + Sal I-digested Ml3mp9 (Figure 8). Plus strand phage DNA is prepared as template for oligonucleotide-directed deletion mutagenesis. Oligonucleotide mut-2 (Table 1) is annealed to the template, and oligonucleotide mut-3 (Table 1) is annealed upstream as second primer. The primers are extended as described above. Oligonucleotide mut-2 directs the fusion of exon II sequences encoding amino acids 23-26 to the cDNA at codon 27. The second primer (mut-3) introduces an Eco RI site 35 bp upstream from the start of translation. The resulting phage are screened for the absence of Nco I and Xho I sites and for the presence of the introduced Eco RI site. Phage DNA showing the desired restriction pattern is sequenced using primer-2 (Table 1) to verify the presence of the correct junction between exon II and the cDNA. Phage DNA with the correct sequence is selected, and the Pst I-Sal I fragment comprising the 5' coding region is isolated from the replicative form of the Ml3 recombinant phage. The fragment is purified by agarose gel electrophoresis and inserted into Pst I + Sal I-digested pUC9 to produce plasmid pC5'end.

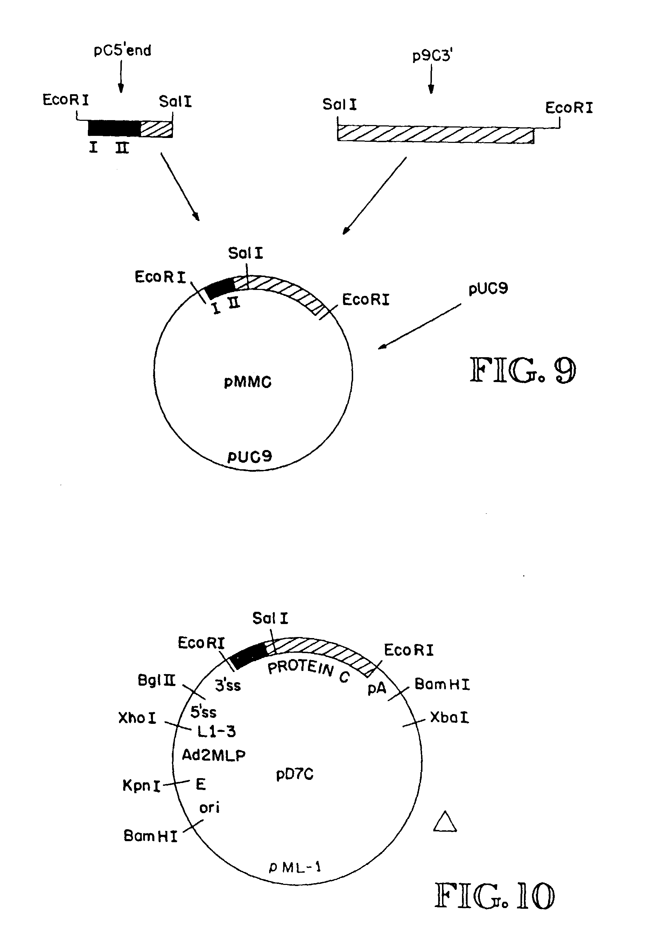

Referring to Figure 9, plasmid pC5'end is digested with EcoRI and Sal I, and the 5' protein C fragment is purified by agarose gel electrophoresis and extraction with CTAB. The remainder of the cDNA is isolated as a Sal I - Eco RI fragment from p9C3'. The two fragments are joined in a three-part ligation to ECO RI-digested pUC9. The ligation mixture is used to transform E. coli JM83, the calls are plated on LB + X-gal, and plasmid DNA is isolated from white colonies. The resultant plasmid is designated pMMC. It contains the complete coding sequence for human protein C on a ca. 1500 bp Eco RI fragment.

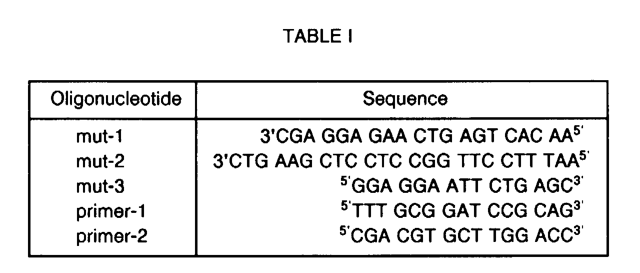

### Example 3

### Construction of Expression Vectors for Protein C

The protein C-encoding insert is removed from pMMC as an Eco RI fragment and inserted into a suitable mammalian cell expression vector. An exemplary vector is pD7, comprising the SV40 enhancer and the adenovirus 2 major late promoter and tripartite leader.

Plasmid pD7 is generated from plasmid pDHFRlll (Berkner and Sharp, Nuc. Acids. Res. 13: 841-857, 1985). The Pst I site immediately upstream from the DHFR sequence in pDHFRlll was converted to a Bcl I site by digesting 10 ug of plasmid with 5 units of Pst I for 10' at 37°C in 100 ul buffer A (10 mM Tris pH 8, 10 mM MgCl₂, 6 mM NaCI, 7mM β-MSH). The DNA was phenol extracted, EtOH precipitated, and resuspended in 40 ul buffer B (50 mM Tris pH 8, 7 mM MgCl₂, 7mM β-MSH) containing 10 mM dCTP and 16 units T₄ DNA polymerase and incubated at 12°C for 60 minutes. Following EtOH precipitation, the DNA was ligated to 2.5 ug kinased Bcl I linkers in 14 ul buffer C (10 mM Tris pH 8, 10 mM MgCl₂, 1 mM DTT, 1.4 mM ATP) containing 400 units T₄ polynucleotide ligase for 12 hours at 12°C. Following phenol extraction and EtOH precipitation, the DNA was resuspended in 120 ul buffer D (75 mM KCI, 6 mM Tris pH 7.5, 10 mM MgCl₂, 1 mM DTT), digested with 80 units Bcl I for 60 minutes at 50°C, then electrophoresed through agarose. Form III plasmid DNA (10 ug) was isolated from the gel, and ligated in 10 ul buffer C containing 50 units T₄ polynucleotide ligase for 2 hours at 12°C, and used to transform E. coli HBl0l. Positive colonies were identified by rapid DNA preparation analysis, and plasmid DNA (designated pDHFR') prepared from positive colonies was transformed into dAM⁻ E. coli.

Plasmid pD2' was then generated by cleaving pDHFR' (15 ug) and pSV40 (comprising Bam HI digested SV40 DNA cloned into the Bam HI site of pML-I) (25 ug) in 100 ul buffer D with 25 units Bcl I for 60 minutes at 50°C, followed by the addition of 50 units Bam HI and additional incubation at 37°C for 60 minutes. DNA fragments were resolved by agarose gel electrophoresis, and the 4.9 kb pDHFR' fragment and 0.2 kb SV40 fragment were isolated. These fragments (200 ng pDHFR' DNA and 100 ng SV40 DNA) were incubated in 10 ul buffer C containing 100 units T₄ polynucleotide ligase for 4 hours at 12°C, and the resulting construct (pD2') was used to transform E. coli RRI.

Plasmid pD2' was modified by deleting the "poison" sequences in the pBR 322 region (Lusky and Botchan, Nature 293: 79-81, 1981). Plasmids pD2' (6.6 ug) and pML-I (Lusky and Botchan, ibid) (4 ug) were incubated in 50 µl buffer A with 10 units each Eco RI and Nru I for 2 hours at 37°C, followed by agarose gel electrophoresis. The 1.7 kb pD2' fragment and 1.8 kb pML-I fragment were isolated and ligated together (50 ng each) in 20 ul buffer C containing 100 units T₄ polynucleotide ligase for 2 hours at 12°C, followed by transformation into E. coli HB101. Colonies containing the desired construct (designated pD2) ware identified by rapid preparation analysis. Ten ug of pD2 were then digested with 20 units each Eco RI and Bgl II, in 50 ul buffer A for 2 hours at 37°C. The DNA was electrophoresed through agarose, and the desired 2.8 kb fragment (fragment C) comprising the pBR322, 3' splice site and poly A sequences was isolated.

To generate the remaining fragments used in constructing pD3, pDHFRlll was modified to convert the Sac II (Sst II) site into either a Hind III or Kpn I Site. Ten ug pDHFRlll ware digested with 20 units Sst II for 2 hours at 37°C, followed by phenol extraction and ethanol precipitation. Resuspended DNA was incubated in 100 ul buffer B containing 10 mM dCTP and 16 units T₄ DNA polymerase for 60 minutes at 12°C, phenol extracted, dialyzed, and ethanol precipitated. DNA (5 ug) was ligated with 50 ng kinased Hind III or Kpn I linkers in 20 ul buffer C containing 400 units T₄ DNA ligase for 10 hours at 12°C, phenol extracted, and ethanol precipitated. After resuspension in 50 ul buffer A, the resultant plasmids were digested with 50 units Hind III or Kpn I, as appropriate, and electrophoresed through agarose. Gel-isolated DNA (250 ng) was ligated in 30 ul buffer C containing 400 units T₄ DNA ligase for 4 hours at 12°C and used to transform E. coli RRI. The resultant plasmids were designated pDHFRlll (Hind III) and pDHFRlll (Kpn I). A 700 bp Kpn T-Bgl II fragment (fragment A) was then purified from pDHFRlll (Kpn I) by digestion with Bgl II and Kpn I followed by agarose gel electrophoresis.

The SV40 enhancer sequence was inserted into pDHFRlll (Hind lll) as follows: 50 ug SV40 DNA was incubated in 120 ul buffer A with 50 units Hind III for 2 hours at 37°C, and the Hind III C SV40 fragment (5089-968 bp) was gel purified. Plasmid pDHFRlll (Hind lll) (10 ug) was treated with 250 ng calf intestinal phosphatase for 1 hour at 37°C, phenol extracted and ethanol precipitated. The linearized plasmid (50 ng) was ligated with 250 ng Hind III C SV40 in 16 ul buffer C for 3 hours at 12°C, using 200 units T₄ polynucleotide ligase, and transformed into E. coli HBl0l. A 700 base pair Eco Rl-Kpn I fragment (fragment B) was then isolated from this plasmid.

For the final construction of pD3, fragments A and B (50 ng each) were ligated with 10 ng fragment C with 200 units T₄ polynucleotide ligase for 4 hours at 12°C, followed by transfection of E. coli RRI. Positive colonies were detected by rapid preparation analysis, and a large-scale preparation of pD3 was made.

Plasmid pD3 is modified to accept the insertion of the protein C sequence by converting the Bcl I insertion site to an Eco RI site. It is first necessary to remove the Eco RI site present in pD3 at the leftmost terminus of the adenovirus 5 0-1 map unit sequences by converting it to a Bam HI site via conventional linkering procedures. Briefly, the plasmid is digested with Eco RI and the linearized DNA treated with T₄ DNA polymerase and all four deoxynucleotide triphosphates to generate blunt termini. The plasmid is then ligated to octonucleotides comprising the Bam HI restriction site, the DNA digested with Bam HI to remove excess linkers, end the fragment comprising the mammalian cell expression sequences is cloned into the Bam HI site of pML-I. The resultant plasmid is transformed into E. coli HB101, and plasmid DNA is prepared and screened for the correct conversion. In a similar manner, the Bcl I site is converted to an Eco RI site using appropriate octonucleotide linkers. The resultant vector is known as pD7. The 1.5 kb protein C Eco RI fragment from pMMC is than inserted into the Eco RI site of pD7 to produce the expression vector pD7C (Figure 10).

A vector enabling expression of the protein C sequence from a polycistronic message is constructed by using pD5, a plasmid similar to pD3 which contains a DHFR coding sequence lacking most of the 5' non-coding region. The DHFR sequence is further modified to reduce its binding affinity to methotrexate.

The vector pD5 is constructed by a method analogous to that described for pD3, and differs from pD3 only in that a Bam HI site is the site of insertion of heterologous DNAs, and that the Bcl I-Bam HT SV40 fragment containing the SV40 polyadenylation signal is in the late orientation.

The DHFR sequence is modified by first digesting pDHFRlll with Pst I and Sst I and isolating the 400 bp DHFR fragment. This is subcloned in an Ml3 phage vector and mutagenized as described by Simonsen and Levinson (Proc. Natl. Acad. Sci. USA 80: 2495-2499, 1983). Mutaganesis results in a single base pair change in the DHFR sequence. The altered fragment is then reinserted into pDHFRlll to produce plasmid pDHFR'III.

The 5' non-coding region of the DHFR sequence is then removed. Plasmid pDHFR^{r}lll is cleaved with Fnu 4HI, which cuts the plasmid at approximately 20 sites, then treated with T₄ DNA polymerase and all four deoxynucleotide triphosphates to generate blunt termini. Bam HI linkers are ligated to the ends, and the mixture digested with Bam HI and Nco I. A 0.6 kb Bam HI-Nco I fragment comprising the DHFR^{r} cDNA is isolated. Plasmid pDHFRlll is digested with Nco I and Bam HI and the 0.2 kb fragment comprising the SV40 polyadenylation signal is isolated. The polyadenylation signal, in the early orientation, is then ligated to the DHFR^{r} fragment. After digestion with Bam HI, the resultant Bam HI fragment is then inserted into the Bam HI site of pD5 and the ligation mixture used to transform E. coli HBl0l. Plasmid DNA is prepared and screened by restriction endonuclease digestion. A plasmid having the DHFR^{r} insert in the correct orientation for transcription from the Ad2 major late promoter is designated pD5(DHFR^{r}).

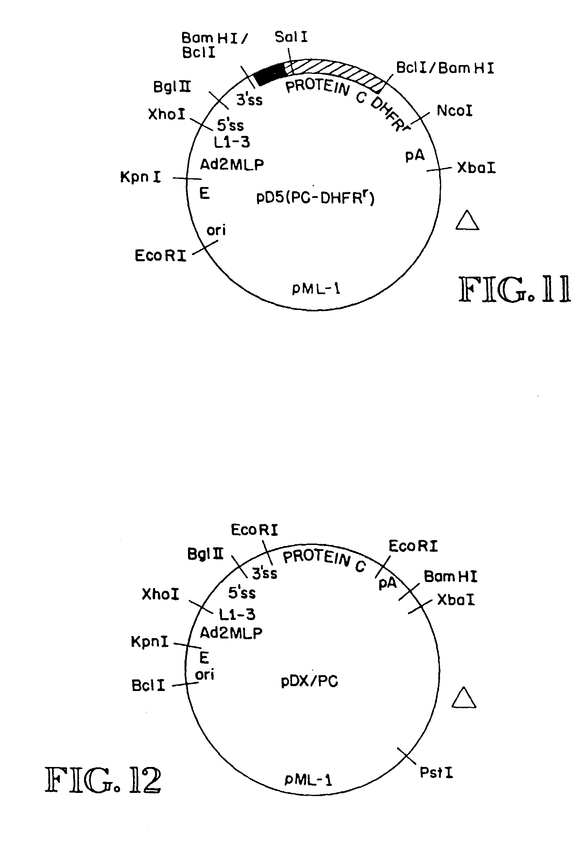

To express protein C using plasmid pD5(DHFR^{r}), pMMC is digested with Eco RI and the 1.5 kb protein C fragment is isolated. The Eco RI termini are converted to Bcl I termini by linkering. Plasmid pD5(DHFR^{r}) is partially digested with Bam HI to cleave it at the 5' end of the DHFR^{r} sequence and is ligated to the protein C fragment. Plasmid DNA is screened for the proper orientation and insertion of the protein C fragment. The resultant vector, designated pD5(PC-DHFR^{r}), is illustrated in Figure 11.

### Example 4

### Expression of Protein C in Transfected Mammalian Cells

Baby hamster kidney (BHK) cells (American Type Culture Collection accession number CCLl0)are transfected with pD7C essentially as described (Wigler et al., Cell 14: 725, 1978; Corsaro and Pearson, Somatic Cell Genetics 7: 603, 1981, and Graham and Van der Eb, Virology 52: 456, 1973). The cells are grown at 37°C, 5% CO₂ in Dulbecco's medium (plus 10% heat-inactivated fetal calf serum and supplemented with glutamine and penicillin-streptomycin) in 60 mm tissue culture Petri dishes to a confluency of 20%. A total of 10 ug of DNA is used to transfect one 60 mm dish: 3.75 ug of pD7C, 1.25 ug of pKO-neo (Southern and Berg, J. Mol. Appl. Genet 1: 327-341, 1982) and 5 ug of salmon sperm DNA. The DNAs are precipitated in 0.3 M WaOAc, 75% ethanol, rinsed with 70% ethanol and redissolved in 20 ul 10 mM Tris-HCl pH8, 1 mM EDTA. The DNA is combined with 440 ul H20 and 500 ul of 280 mM Nacl, 1.5 mM NaHPO₄, 12 mM dextrose, 50 mM HEPES pH 7.12. Sixty ul of 250 mM CaCl₂ are added dropwise to the above mixture and the solution let stand at room temperature for 30 minutes. The solution is then added to the cells and the cells returned to 37°C for 4 hours. The medium is removed and 5 ml of 20% DMSO in Dulbecco's with serum are added for 2 minutes at room temperature. The dish is then washed rapidly with 2 changes of medium and incubated in fresh medium overnight. Twenty-four hours after the addition of the DNA, the medium is removed and selective medium (10 mg/ml of G418, 498 u/mg, Gibco, in Dulbecco's with serum) added. After approximately 10-13 days, individual clones, representing cells that have incorporated the pKO-neo gene and are thus resistant to G418, are transferred to 96-well plates and grown up for protein assays in Dulbecco's plus 10% fetal calf serum.

To assay for protein C, the medium is separated from the cells and cellular debris by centrifugation, and assayed for protein C polypeptide and biological activity. The cells are removed from the plates with trypsin, washed with fresh medium, centrifuged and frozen at -20°C. For assay, the cell pellets are thawed in PBS, pelleted, and resuspended in PBS containing 0.25% Triton® X-100. Samples are diluted and assayed for polypeptide and activity.

The ELISA for protein C is done as follows: Two hundred microliters of antibody (monoclonal or, polyclonal) against human protein C (5 ul/ml in 0.1 M Na₂CO₃ pH 9.6) are incubated in each well of a 96-well microtiter plate 2 hours at 37°C. The wells are then incubated with 220 ul of 1% bovine serum albumin (BSA) and 0.05% Tween® 20 in PBS pH 7.2 for 2 hours at 37°C. The plates are rinsed with H₂O, air dried, and stored at 4°C. To assay samples, 200 ul samples are incubated 1 hour at room temperature in the antibody-coated wells. The wells are then rinsed four times with 200 ul PBS containing 0.05% Tween® 20. The wells are then incubated for 1 hour at room temperature with 200 ul of an lgG fraction of rabbit polyclonal antiserum against protein C (5 ug/ml in PBS containing 1% BSA and 0.05% Tween 20). This is followed by incubation with goat anti-rabbit lgG coupled to alkaline phosphatase. The wells are then rinsed four times with PBS containing 0.05% Tween® 20. To the wells are added 200 ul p-nitrophenyl phosphate (30 mg) dissolved in diethanolamine buffer (96 ml per liter) pH 9.8 containing 56 mg/l MgCl₂. The enzyme reaction is done at 37°C and the development of a yellow color is monitored at 405 nm using an ELISA plate reader.

Protein C biological activity is assayed by its ability to prolong the kaolin-cephalin clotting time of plasma following its activation by thrombin as described by Kisiel and Davie (Meth. in Enzymology 80: 320-332, 1981).

### Example 5

### Expression of a Full-Length cDNA Encoding Protein C

A. Isolation of cDNA. A genomic fragment containing an exon corresponding to amino acids -42 to -19 of the pre-pro peptide (Exon 1 in Figure 4) of protein C was isolated, nick translated, and used as a probe to screen a cDNA library constructed by the technique of Gubler and Hoffman (Gene 25:263-269, 1983) using mRNA from HepG2 cells. This cell line was derived from human hepatocytes and was previously shown to synthesize protein C (Fair and Bahnak, Blood 64: 194-204, 1984). Ten positive clones comprising cDNA inserted into the Eco RI site of phage λgtll were isolated and screened with an oligonucleotide probe corresponding to the 5' noncoding region of the protein C gene. One clone was also positive with this probe and its entire nucleotide sequence was determined. The cDNA contained 70 bp of 5' untranslated sequence, the entire coding sequence for human prepro-protein C, and the entire 3' noncoding region corresponding to the second polyadenylation site (Figure 2).
B. Expression Vector Construction. The expression of protein C cDNA was achieved in the vector pDX. This vector was derived from pD3 (described in Example 3 above) and pD3', a vector identical to pD3 except that the SV40 polyadenylation signal (i.e. the SV40 BamHl [2533 bp] to Bcll [2770 bp] fragment) is in the late orientation. Thus, pD3' contains a Bam HI site as the site of gene insertion.
   To generate pDX, the Eco RI site in pD3' was converted to a Bcll site by Eco RI cleavage, incubation with Sl nuclease, and subsequent ligation with Bcl I linkers. DNA was prepared from a positively identified colony, and the 1.9 kb Xho l-Pst I fragment containing the altered restriction site was prepared via agarose gel electrophoresis. In a second modification, Bcl I-cleaved pD3 was ligated with kinased Eco Rl-Bcl I adaptors (constructed from oligonucleotides ZC 525, 5'GGAATTCT3'; and ZC526, 5'GATCAGAATTCC3') in order to generate an Eco RI site as the position for inserting a gene into the expression vector. Positive colonies were identified by restriction endonuclease analysis, and DNA from this was used to isolate a 2.3 kb Xho l--Pst I fragment containing the modified restriction site. The two above-described DNA fragments were incubated together with T4 DNA ligase, transformed into E. coli HBl0l and positive colonies were identified by restriction analysis. A preparation of such DNA, termed pDX, was then made. This plasmid contains a unique Eco RI site for insertion of foreign genes.
   The protein C cDNA was then inserted into pDX as an Eco RI fragment. Recombinant plasmids were screened by restriction analysis to identity those having the protein C insert in the correct orientation with respect to the promoter elements and plasmid DNA (designated pDX/PC) was prepared from a correct clone (Figure 12). Because the cDNA insert in pDX/PC contains an ATG codon in the 5' noncoding region (see Figure 2), deletion mutagenesis was performed on the cDNA prior to transfection and expression experiments. Deletion of the three base pairs was performed according to standard procedures of oligonucleotide-directed mutagenesis. The pDX-based vector containing the modified cDNA was designated p594.
C. cDNA Expression. Plasmid p594 was transfected into COS cells by calcium phosphate precipitation. Four hours later, fresh culture media (supplemented with 5 ug/ml vitamin K) was added. At appropriate times (usually 48 or 72 hours), the culture media were harvested and the cells were collected and lysed.

The protein C secreted into the culture medium or the cell extracts was assayed by ELISA using the same affinity - purified polyclonal antibody which was used in the initial identification of the cDNA clones. Results of the assays (Table 2) showed that protein C was synthesized in the experimental samples and was readily secreted from the transfected cells, with approximately 90% of the protein C found in the culture media.

To assess the extent of gamma-carboxylation of the recombinant protein, samples of the culture media were subjected to barium citrate precipitation, a process which selectively precipitates only gamma-carboxylated proteins from plasma (Bajaj et al., J. Biol. Chem. 256: 253-259, 1981). Over 70% of the antigenic material could be precipitated with barium citrate.

The recombinant protein C was assayed for anticoagulant activity by measuring its ability to prolong coagulation. Dialyzed media samples were treated with Protac C (American Diagnostica) to activate the protein C. The samples were then added to an in vitro clotting assay (Sugo et al., J. Biol. Chem. 260:10453, 1985) and the clotting time was measured. The activity of the recombinant material was shown to be essentially the same as that of naturally occurring protein C.

**TABLE 2**

| TRANSIENT EXPRESSION AND SECRETION OF PROTEIN C IN COS CELLS | | | |
|---|---|---|---|
| plasmid | ng protein C in media | ng protein C cell extract | % secreted |
| none | 0 | 0 | NA |
| p594 | 165 | 20 | 89% |

From the foregoing it will be appreciated that, although specific embodiments of the invention have been described herein for purposes of illustration, various modifications may be made without deviating from the scope of the invention. Accordingly, the invention is not to be limited except as by the appended claims.

The features disclosed in the foregoing description, in the following claims and/or in the accompanying drawings may, both separately and in any combination thereof, be material for realising the invention in diverse forms thereof.

## Claims

1. A DNA sequence which codes for a protein which upon activation by proteolytic processing in the secretion pathway results in a human activated protein C, said DNA sequence not including a sequence encoding the twelve amino acid activation peptide shown in Figure 2, said activated protein C having an amino acid sequence shown in Figure 2.

2. A recombinant expression vector comprising a DNA sequence which codes for a protein which upon activation by proteolytic processing in the secretion pathway results in a human activated protein C, said DNA sequence not including a sequence encoding the twelve amino acid activation peptide shown in Figure 2, said activated protein C having an amino acid sequence shown in Figure 2.

3. An expression vector capable of integration in mammalian host cell DNA, said expression vector including a promoter followed downstream by a nucleotide sequence of claim 2, said nucleotide sequence being followed downstream by a polyadenylation signal, wherein transcription of the nucleotide sequence is directed by the promoter.

4. A method for producing human activated protein C, comprising:
introducing into a mammalian host cell an expression unit comprising a sequence of claim 1, which encodes a protein which upon activation by intracellular proteolytic processing results in a human activated protein C;
growing said mammalian host cell in an appropriate medium which contains vitamin K; and
isolating the protein product encoded by said expression unit and produced by said mammalian host cell.

5. A method according to claim 4, further comprising introducing into the host cell, with said expression unit, a selectable marker.

6. A method according to claim 4, wherein said expression unit is contained on an expression vector capable of integration in mammalian host cell DNA, said expression vector including a promoter followed downstream by a nucleotide sequence which encodes human activated protein C, said nucleotide sequence being followed downstream by a polyadenylation signal, wherein transcription of the nucleotide sequence is directed by the promoter.

7. A method according to claim 4 wherein said mammalian host cell is a baby hamster kidney cell or a COS cell.

8. A DNA sequence which codes for a protein having human protein C activity or a protein which upon activation by intracellular proteolytic processing results in a protein having a human activated protein C biological activity, wherein said protein further comprises an amino-terminal calcium-binding domain of a protein selected from the group consisting of factor VII, factor IX, factor X and prothrombin.

9. Mammalian cells transfected with an expression vector according to claim 2 or claim 3 to express human activated protein C.

## Patentansprüche

1. Eine DNA-Sequenz, die für ein Protein kodiert, das bei Aktivierung durch proteolytische Verarbeitung im Sekretionsweg zu einem menschlichen aktivierten Protein C führt, wobei besagte DNA-Sequenz nicht eine Sequenz einschließt, die das in Fig. 2 dargestellte Aktivierungspeptid aus zwölf Aminosäuren kodiert, wobei besagtes aktiviertes Protein C eine Aminosäuresequenz aufweist, die in Figur 2 dargestellt ist.

2. Ein rekombinanter Expressionsvektor, der eine DNA-Sequenz umfaßt, die für ein Protein kodiert, das bei Aktivierung durch proteolytische Verarbeitung im Sekretionsweg zu einem menschlichen aktivierten Protein C führt, wobei besagte DNA-Sequenz nicht eine Sequenz einschließt, die das in Figur 2 dargestellte Aktivierungspeptid aus zwölf Aminosäuren kodiert, wobei besagtes aktiviertes Protein C eine Aminosäuresequenz aufweist, die in Figur 2 dargestellt ist.

3. Ein Expressionsvektor, der zur Integration in Säugetier-Wirtszell-DNA in der Lage ist, wobei besagter Expressionsvektor einen Promotor einschließt, dem stromabwärts eine Nukleotidsequenz von Anspruch 2 folgt, wobei besagter Nukleotidsequenz stromabwärts ein Polyadenylierungssignal folgt, wobei die Transkription der Nukleotidsequenz durch den Promotor gesteuert wird.

4. Ein Verfahren zur Herstellung von menschlichem aktivierten Protein C, welches umfaßt:
Einführen einer Expressionseinheit, die eine Sequenz von Anspruch 1 umfaßt, die ein Protein kodiert, das bei Aktivierung durch intrazelluläre proteolytische Verarbeitung zu einem menschlichen aktivierten Protein C führt, in eine Säugetier-Wirtszelle;
Züchten besagter Säugetier-Wirtszelle in einem geeigneten Medium, das Vitamin K enthält;
und Isolieren des Proteinproduktes, das durch besagte Expressionseinheit kodiert und durch besagte Säugetier-Wirtszelle produziert wird.

5. Ein Verfahren nach Anspruch 4, welches außerdem das Einführen, mit besagter Expressionseinheit, eines selektionierbaren Markers in die Wirtszelle umfaßt.

6. Ein Verfahren nach Anspruch 4, wobei besagte Expressionseinheit auf einem Expressionsvektor enthalten ist, der zur Integration in Säugetier-Wirtszell-DNA in der Lage ist, wobei besagter Expressionsvektor einen Promotor einschließt, dem stromabwärts eine Nukleotidsequenz folgt, die menschliches aktiviertes Protein C kodiert, wobei besagter Nukleotidsequenz stromabwärts ein Polyadenylierungssignal folgt, wobei die Transkription der Nukleotidsequenz durch den Promotor gesteuert wird.

7. Ein Verfahren nach Anspruch 4, wobei besagte Säugetier-Wirtszelle eine Babyhamster-Nierenzelle oder eine COS-Zelle ist.

8. Eine DNA-Sequenz, die für ein Protein mit der Aktivität von menschlichem Protein C oder ein Protein, das bei Aktivierung durch intrazelluläre proteolytische Verarbeitung zu einem Protein mit der biologischen Aktivität eines menschlich aktivierten Proteins C führt, kodiert, wobei besagtes Protein außerdem eine aminoterminale Calciumbindungsdomäne eines Proteins umfaßt, das ausgewählt ist aus der Gruppe, die aus Faktor VII, Faktor IX, Faktor X und Prothrombin besteht.

9. Säugetierzellen, die mit einem Expressionsvektor nach Anspruch 2 oder Anspruch 3 transfiziert sind, um menschliches aktiviertes Protein C zu exprimieren.

## Revendications

1. Séquence d'ADN qui code pour une protéine qui, par activation par maturation protéolytique dans la voie métabolique de sécrétion, a pour résultat une protéine C humaine activée, ladite séquence d'ADN ne comprenant pas de séquence codant pour le peptide d'activation de 12-amino-acides représenté sur la figure 2, ladite protéine C activée ayant une séquence d'amino-acides représentée sur la figure 2.

2. Vecteur d'expression recombinant comprenant une séquence d'ADN qui code pour une protéine qui, par activation par maturation protéolytique dans la voie métabolique de sécrétion, a pour résultat une protéine C humaine activée, ladite séquence d'ADN ne comprenant pas de séquence codant pour le peptide d'activation de 12-amino-acides représenté sur la figure 2, ladite protéine C activée ayant une séquence d'amino-acides représentée sur la figure 2.

3. Vecteur d'expression apte à l'intégration dans l'ADN d'une cellule hôte de mammifère, ledit vecteur d'expression comprenant un promoteur suivi, en aval, par une séquence de nucléotides suivant la revendication 2, ladite séquence de nucléotides étant suivie, en aval, d'un signal de polyadénylation, dans lequel la transcription de la séquence de nucléotides est dirigée par le promoteur.

4. Procédé pour la production de protéine C humaine activée, comprenant :
l'introduction dans une cellule hôte de mammifère d'une unité d'expression comprenant une séquence suivant la revendication 1, qui code pour une protéine qui, par activation par maturation protéolytique intracellulaire, a pour résultat une protéine C humaine activée ;
la culture de ladite cellule hôte de mammifère dans un milieu approprié qui contient de la vitamine K ; et
l'isolement du produit protéique codé par ladite unité d'expression et élaboré par la cellule hôte de mammifère.

5. Procédé suivant la revendication 4, comprenant en outre l'introduction dans la cellule hôte, avec l'unité d'expression, d'un marqueur sélectionnable.

6. Procédé suivant la revendication 4, dans lequel l'unité d'expression est présente sur un vecteur d'expression apte à l'intégration dans l'ADN d'une cellule hôte de mammifère, ledit vecteur d'expression comprenant un promoteur suivi, en aval, par une séquence de nucléotides qui code pour la protéine C humaine activée, ladite séquence de nucléotides étant suivie, en aval, par un signal de polyadénylation, où la transcription de la séquence de nucléotides est dirigée par le promoteur.

7. Procédé suivant la revendication 4, dans lequel la cellule hôte de mammifère est une cellule rénale de hamster nouveau-né ou une cellule COS.

8. Séquence d'ADN qui code pour une protéine ayant l'activité de la protéine C humaine ou une protéine qui, par activation par maturation protéolytique intracellulaire, a pour résultat une protéine ayant une activité biologique de la protéine C humaine activée, ladite protéine comprenant en outre un domaine de fixation de calcium aminoterminal d'une protéine choisie dans le groupe consistant en le facteur VII, le facteur IX, le facteur X et la prothrombine.

9. Cellules de mammifère transfectées avec un vecteur d'expression suivant la revendication 2 ou la revendication 3 pour l'expression de la protéine C humaine activée.
